# EUROPÄISCHE PATENTSCHRIFT

(11) **EP 2 579 815 B1**
(45) Veröffentlichungstag und Bekanntmachung des Hinweises auf die Patenterteilung: **05.04.2017**
(21) Anmeldenummer: 11724563.9
(22) Anmeldetag: 10.06.2011
(51) Int. Cl.: A61F 2/28, A61F 2/30, A61F 2/36, A61F 2/38

(54) **PROTHESE ZUM TEILWEISEN ERSATZ EINES RÖHRENKNOCHENS**
PROSTHETIC FOR PARTIAL REPLACEMENT OF A LONG BONE
PROTHÈSE POUR LE REMPLACEMENT PARTIEL D'UN OS LONG

(30) Priorität: 11.06.2010 EP 10006098
(43) Veröffentlichungstag der Anmeldung: 17.04.2013
(73) Patentinhaber: Waldemar Link GmbH & Co. KG, 22315 Hamburg (DE)
(72) Erfinder: LINK, Helmut, D., 22397 Hamburg (DE); DÄNIKE, Andreas, 22339 Hamburg (DE); JENDRO, Günther, 22339 Hamburg (DE)
(74) Vertreter: Hoffmann Eitle
(86) Internationale Anmeldenummer: PCT/EP2011/002875
(87) Internationale Veröffentlichungsnummer: WO 2011/154156

(56) Entgegenhaltungen:
- EP-A1- 0 290 767
- EP-A1- 0 490 159
- EP-A1- 0 621 019
- DE-A1- 3 336 004
- FR-A1- 2 666 221
- US-A- 4 502 160
- US-A1- 2003 149 486

## Beschreibung

Die Erfindung betrifft eine Prothese zum mindestens teilweisen Ersatz eines Röhrenknochens und eines angrenzenden Gelenks. Sie umfasst einen länglich gestreckten Schaft mit einem ersten und einem zweiten Ende, sowie eine Gelenkeinrichtung, die am zweiten Ende des Schafts angeordnet ist. Es ist eine Längeneinstellrichtung vorgesehen, welche den Schaft längs seiner Achse teleskopartig verschiebt. Die Erfindung erstreckt sich weiter auf ein Prothesenmodülsystem mit auswechselbaren Schäften.

Zum Ersatz von erkrankten oder defekten Knochen und Gelenken sind verschiedene Typen von Endoprothesen seit langem bekannt. Zum Ersatz von Röhrenknochen, insbesondere aufgrund von Tumorerkrankungen, werden Prothesen verwendet, die einen sich über die Länge des zu ersetzenden Knochens ausgedehnten Schaft aufweisen. Der Schaft ersetzt bzw. verstärkt den erkrankten oder fehlenden Bereich des Knochens. Häufig ist er verbunden mit einer Gelenkeinheit, welche ein angrenzendes Gelenk (zum Beispiel Knie oder Ellenbogen) ersetzt. Die Abmessungen des Prothesenschafts müssen daher entsprechend der jeweiligen Anatomie und Pathologie der Patienten gewählt sein.
Zur Anpassung an die individuellen Bedürfnisse ist es bekannt, die Prothesen in verschiedenen Größen anzubieten. Jedoch kann selbst mit einer feinen Abstufung keine optimale Versorgung in Anbetracht der Vielzahl an verschiedenen Bedürfnissen erreicht werden. Dies gilt noch viel mehr bei Patienten, die sich in der Wachstumsphase befinden, also Kindern. Um auch bei solchen Patienten eine ausreichende Versorgung zu ermöglichen, sind Prothesen mit einer Längeneinstelleinrichtung versehen worden. So ist eine Knieprothese bekannt, die einen Schaft und eine Gelenkeinrichtung umfasst, wobei im Schaft eine Teleskopeinrichtung zur Veränderung der Schaftlänge vorgesehen ist (US 4,384,373). Hierbei ist eine Längeneinstellung des Schafts im Rahmen der Operation ermöglicht. Eine nachträgliche Verstellung ist nicht vorgesehen

Um auch post-operativ die Länge des Schafts einstellen zu können, ist eine weiterentwickelte Prothese bekannt (US 4,502,160), bei der zur Betätigung eine Überwurfmutter vorgesehen ist. Sie weist einen Außenzahnkranz auf, der über einen von lateral anzusetzenden Steckschlüssel mit einer Außenverzahnung betätigbar ist. Der Steckschlüssel kann durch eine Inzision geführt sein, so dass auch nach der Operation die Länge des Schafts verstellt werden kann.

Um auch bei der Veränderung der Länge eine Rotation der Prothese, insbesondere eine Rotation von Schaft relativ zum Gelenk, zu vermeiden, kann eine Rotationssperre vorgesehen sein (US 4,892,546). Mittels einer Sperrschraube ist der Schaft an einer Verdrehung gegenüber dem Gelenk gehindert, wobei zur Längenverstellung die Sperrschraube gelöst ist.

Des Weiteren offenbart die DE 33 36 004 A1 eine Endoprothese mit einem Gelenkteil und einem Schaft für im Wachstum befindliche Menschen, wobei der Gelenkteil und Schaft über einen in der Länge veränderbaren Adapter lösbar in Verbindung stehen. Der Adapter besteht aus zwei zueinander verdrehbaren, im Wesentlichen zylindrischen mit dem Gelenkteil und dem Schaft lösbar verbundenen Hülsen, von denen die eine eine axial unbewegliche Verlängerung eines verdrehbaren Kopfes einer Schraube aufnimmt, die in ein Innengewinde der anderen Hülse einschraubbar ist.

Ein Nachteil dieser bekannten Prothesen liegt darin, dass sie jeweils sehr spezialisiert (post-operativ betätigbar, rotationsgeschützt etc.) sind, und deshalb jeweils nur ein schmales Anwendungsfeld haben.
Der Erfindung liegt die Aufgabe zugrunde, eine Endoprothese der eingangs genannten Art dahingehend weiterzuentwickeln, dass sie universeller einsetzbar ist.

Die erfindungsgemäße Lösung liegt in den Merkmalen des Anspruchs 1. Vorteilhafte Weiterbildungen sind Gegenstand der abhängigen Ansprüche.
Bei einer Prothese zum mindestens teilweisen Ersatz eines Röhrenknochens und eines angrenzenden Gelenks, umfassend einen länglich gestreckten Schaft mit einem ersten und einem zweiten Ende, eine Gelenkeinrichtung, die am zweiten Ende des Schafts angeordnet ist, wobei eine Längeneinstellrichtung vorgesehen ist, welche den Schaft längs seiner Achse teleskopartig betätigt, ist erfindungsgemäß vorgesehen, dass der Schaft und die Gelenkeinrichtung über komplementäre Steckverbinder gekoppelt sind, wobei die Längeneinstelleinrichtung modular ausgeführt ist und an ihrem proximalen und distalen Ende mit den komplementären Steckverbindern versehen ist, und sie weiter mit einer formschlüssig wirkenden Verdrehsicherung versehen ist. Unter komplementär wird verstanden, dass am einen der beiden Enden ein männlicher und am anderen ein weiblicher Steckverbinder vorgesehen sind. Die Steckverbinder sind Konussteckverbinder.
Kern der Erfindung ist der Gedanke, die Längeneinstelleinrichtung modular auszugestalten und sie dazu an ihrem proximalen und distalen Ende mit genau solchen Steckverbindern zu versehen, die auch am Übergang zwischen Schaft und Gelenk der Prothese angeordnet sind. Die Längeneinstelleinrichtung ist also anders als im Stand der Technik nicht integraler Bestandteil der Prothese, sondern kann nach Bedarf eingesetzt sein. Sie ist sozusagen austauschbar gegen ein herkömmliches Standard-Schaftelement ohne Längeneinstelleinrichtung.

Die Erfindung ermöglicht es damit auf einfache und effiziente Weise, an sich beliebige Gelenkprothesen mit einer Längeneinstelleinrichtung zu versehen. Die Anpassbarkeit der Prothese an die anatomischen bzw. pathologischen Verhältnisse des einzelnen Patienten verbessert sich damit erheblich, und zwar ohne dass dafür eine Vielzahl von verschiedenen Teilen in unterschiedlicher Größe erforderlich wäre. Die Gelenkeinrichtung kann an sich beliebig ausgeführt sein, wobei sie Bewegungen im Gelenk in unterschiedlichem Grad stützen oder beschränken kann von völlig frei bis zu versteift. Indem die Längeneinstelleinrichtung erfindungsgemäß von der eigentlichen Gelenkprothese getrennt ist dank der modularen Ausführung, kann die Erfindung ohne weiteres auch an anderen Prothesen angewendet sein, solange sie nur über entsprechende Konussteckverbinder verfügen. Dank der integrierten formschlüssig wirkenden Verdrehsicherung sind keine weiteren Anforderungen in Bezug auf Verdrehschutz an die jeweilige Grundprothese gestellt.

Die Verdrehsicherung hindert eine unerwünschte relative Verdrehung des Schafts und seiner Komponenten. Die bauliche Integration von Verdrehsicherung und Längeneinstellung hat weiter den Vorteil, dass die Betätigungselemente eng beieinander liegen können. Damit ist zur post-operativen Verstellung nur ein Zugang an einer räumlich eng umgrenzten Stelle erforderlich. Es genügt eine nur minimal invasive Stichinzision, um die Prothese in ihrer Länge zu verändern. Mit einer solch schonenden Operationstechnik ist die Prothese insbesondere auch zur Verwendung bei Kindern geeignet.

Vorzugsweise weist der Schaft einen Außen- und einen Innenstab auf, auf welchen die Längeneinstelleinrichtung wirkt. Damit kann mittels eines geeigneten Werkzeugs unmittelbar auf die Längeneinstelleinrichtung eingewirkt werden, die entsprechend den Außenstab teleskopartig gegenüber dem Innenstab verschiebt.

Um trotz der einfachen Verstellbarkeit einen ausreichenden Schutz vor unbeabsichtigter Verstellung zu gewährleisten, ist vorzugsweise eine Doppelsicherung vorgesehen, welche zusätzlich zur Verdrehsicherung eine Verstellsicherung mittels zweier benachbarter Schrauben bildet. Hierbei ist weiter vorzugsweise die eine drehfest am Außenstab und die andere drehbeweglich an der Verstellmutter angeordnet.

Der Außenstab weist mit Vorteil einen Druckflansch auf, der zwei einander gegenüberliegende Bundflächen aufweist, von denen die eine ein Schublager für die Konusverbindung und die andere einen Anschlag für die Längeneinstellung bildet. Damit ist ein sehr kompakter Aufbau ermöglicht, der eine Integration der erfindungsgemäßen Längeneinstelleinrichtung auch an verhältnismäßig kleine Prothesen ermöglicht, beispielsweise zur Anwendung am Ellbogen oder an der Hand.

Es kann zweckmäßig sein, wenn eine zweite Längeneinstelleinrichtung für den Schaft vorgesehen ist, die vorzugsweise mit invers angeordneten Konusverbindern versehen ist. Bei langen Schäften, insbesondere solchen zum Ersatz des Femurs, kann damit auch am anderen Ende eine Längeneinstellung erfolgen. Dies vergrößert nicht nur den Einstellbereich, sondern ist häufig auch physiologisch günstiger.

Die Erfindung erstreckt sich weiter auf ein Prothesensystem mit mehreren koppelbaren verschieden langen, insbesondere starren, Schaftelementen, die über die Steckverbinder koppelbar sind, wobei vorzugsweise mindestens eines der insbesondere starren Schaftelemente dieselbe Länge wie die Längeneinstelleinrichtung in ihrer Grundstellung aufweist. Damit können innerhalb eines Prothesensystems solche Prothesen gebildet sein, die einen Schaft mit starrer Länge oder einen Schaft mit einstellbarer Länge aufweisen, wobei durch einfachen Austausch eines starren Schaftmoduls gegen eines mit einstellbarer Länge hin- und hergewechselt werden kann. Dies kann auch intraoperativ erfolgen, so dass je nach Lage des Falls der Chirurg noch im Laufe der Operation entscheiden kann, welche Variante im jeweiligen Fall vorzugsweise verwendet werden soll.

Gemäß einer besonders vorteilhaften Variante, die gegebenenfalls unabhängigen Schutz verdient, ist bei einer Prothese zum mindestens teilweisen Ersatz eines Röhrenknochens vorgesehen, dass eine Betätigungseinrichtung für die Längeneinstelleinrichtung mit einem Gewinde auf dem Innenstab und einer auf das Gewinde geschraubte Verstellmutter mit einer umlaufenden Verzahnung vorgesehen ist, wobei am Außenstab eine Lagerbohrung für einen in die umlaufende Verzahnung greifenden Verstellschlüssel vorgesehen ist. Vorzugsweise ist vorgesehen, dass die Verstellmutter mit ihrem oberen Rand abhebbar auf einer Stirnfläche des Außenstabs aufliegt und hinterschnittfrei mit ihm zusammen wirkt.

Kern dieses Erfindungsaspekts ist der Gedanke mittels der Lagerbohrung für den Verstellschlüssel mit einer sehr kleinen und patientenschonenden Zugangsöffnung auszukommen. Damit kann die Länge häufig nachgestellt und - vor allem bei jungen Patienten - dem Wachstum angepasst werden. Dank der modulären Konstruktion ist ein Austausch der Längeneinstelleinrichtung leicht möglich, um - wenn die Verstellung ausgeschöpft ist - eine größere einzusetzen.

Dank der vorzugsweise frei gelagerten Verstellmutter ist eine axiale Verschieblichkeit gegenüber dem Außenstab gegeben, nämlich indem die Verstellmutter an dessen Stirnseite nur aufliegt, ohne dort mittels einer formschlüssigen Führung, insbesondere einem Hinterschnitt, gesichert zu sein; die Verstellmutter kann damit von dem Außenstab frei wegbewegt werden.

Mit dieser Konstruktion werden zwei wesentliche Vorteile verknüpft. Zum einen wird damit erreicht, dass die Prothesenteile voneinander getrennt werden können. Die zur Implantation der Röhrenknochenprothese erforderliche Wundöffnung kann damit kleiner ausfallen. Für den Patienten ist dies deutlich schonender, und für den Chirurgen ist das leichter zu handhaben.

Ein weiterer Vorteil besteht darin, dass bedingt durch das Merkmal der hinterschnittfreien Lagerung der Verstellmutter eine größere Kraftauflagefläche zwischen Verstellmutter und dem Außenstab an der Stirnseite ermöglicht ist. Die Prothese ist dank der größeren Kraftauflagefläche somit weniger belastet bzw. sie kann bei gleicher Robustheit kleiner und damit schlanker ausgeführt sein. Gerade letzteres bietet einen erheblichen Vorteil zur Implantation bei jungen Patienten.

Zwar ist es bekannt, bei einer Prothese zum Ersatz eines Röhrenknochens mit angrenzendem Gelenk in dem Übergang zwischen Gelenk und Röhrenknochen ein Kegelradgetriebe zur Längenverstellung des Röhrenknochenersatzes vorzusehen (US 4,892,546). Dies bietet zwar den Vorzug, dass eine Längenverstellung ohne großen operativen Eingriff erfolgen kann. Ein Nachteil besteht jedoch darin, dass das erforderliche Kegelradgetriebe verhältnismäßig klobig ist. Die Prothese eignet sich daher weniger zur Anwendung bei jungen Patienten, insbesondere Kindern. Weiter ist eine Röhrenknochenprothese mit einem Teleskopschaft bekannt, der einen Schaft und eine Hülse umfasst, wobei an der Hülse eine Überwurfmutter vorgesehen ist (US 4, 502,160). Die Überwurfmutter ist in Axialrichtung durch eine formschlüssige Führung fest an der Hülse geführt, so dass sie sich nur verdrehen, nicht aber in Längsrichtung bewegen kann. Die Überwurfmutter wirkt mit ihrem inneren Gewinde mit einem auf dem Schaft angeordneten Außengewinde zusammen. Durch Verdrehen der Überwurfmutter kann die Länge verändert werden. Bedingt durch die formschlüssige Fixierung der Überwurfmutter an der Hülse kann eine Implantation der Prothese ausschließlich in voll montiertem Zustand erfolgen. Dies verkompliziert die Implantation, da für die Prothese im voll montierten Zustand eine große Zugangsöffnung erforderlich ist. Die Operationswunde wird damit unverhältnismäßig groß, was gerade für die Gruppe der jungen Patienten eine schwere Belastung darstellen kann.

Die erfindungsgemäße Prothese ist damit wesentlich schonender für den Patienten und günstiger in Bezug auf das Wachstumsverhalten, und ist somit insbesondere zur Behandlung von jungen Patienten (Kindern) in der Wachstumsphase geeignet. Denn bei der Implantation muss häufig die Wachstumslage des Knochens reseziert werden. Sie ist aber auch durchaus geeignet zur Anwendung bei Erwachsenen, wenn sich post-operativ Veränderungen ergeben, beispielsweise auf Grund von Längungen im Bandapparat.

Die umlaufende Verzahnung ist vorzugsweise als Steilverzahnung ausgeführt. Unter Steilverzahnung wird verstanden, dass die lasttragenden Flanken einen Flankenwinkel von mindestens 50° bis höchstens 85°, vorzugsweise mindestens 60° aufweisen. Mit einer solchen steilen Ausrichtung der Last tragenden Flanken wird die Ausbildung einer Axialkraft durch das Betätigen des Verstellschlüssels und dessen Einwirkung auf die umlaufende Verzahnung der Verstellmutter unterdrückt bzw. weitgehend vermieden. Eine unerwünschte parasitäre Längenverstellung oder eine unerwünschte axiale Verschiebung auf Grund des Verstellschlüssels kann damit vermieden werden. Es ist somit sichergestellt, dass die Längenverstellung allein aus der über die Drehbewegung der Verstellmutter übertragenen Axialverschiebung auf Grund der Gewindesteigung des Innengewindes der Verstellmutter beruht.

Vorzugsweise ist die Verzahnung in einem entsprechend umlaufenden Rezess eingebettet. Der Rezess ist dabei vorzugsweise an der Außenkante der Oberseite ausgebildet. Damit wird erreicht, dass die umlaufende Verzahnung nicht übersteht, also dass keine Spitzen in Axialrichtung vorragen.

Damit wird der Gefahr einer Irritation umliegenden Gewebes auf wirksame Weise begegnet.

Für alle Ausführungsformen gilt außerdem:

Mit Vorteil ist das Innengewinde der Verstellmutter eingängig. Unter "eingängig" wird hierbei verstanden, dass es nur einen Gewindegang gibt, der von einer Seite der Mutter zur gegenüberliegenden Seite durchläuft. Mit der Eingängigkeit wird eine definierte Positionierung der Verstellmutter in Drehrichtung in Bezug auf den Innenstab erreicht. Dies erleichtert eine präzise Ausrichtung und damit Längenverstellung. Die Gefahr von positionsmäßigen Doppeldeutigkeiten besteht damit nicht.

Vorzugsweise ist das Gewinde des Innenstabs geplant. Unter "geplant" wird hierbei verstanden, dass die Spitzen des Gewindes an dem Innenstab gebrochen sind, also nicht im eigentlichen Sinne spitz sind, sondern durch eine vorzugsweise ebene Fläche ersetzt sind. Diese ebene Fläche bildet in ihrer Gesamtheit einen Hohlzylindermantel. Das Gewinde des Innenstabs wirkt damit gegenüber seiner Umgebung weniger scharfkantig. Die Gefahr von Irritationen wird damit verringert.

Mit Vorteil weist die Verstellmutter eine polierte Umfangsfläche auf. Hiermit wird eine negative Wirkung des umliegenden Gewebes auf die Verstellmutter vermieden, so dass es kaum zu Anhaftungen kommt. Die Verstellmutter bleibt damit auch nach mehrjähriger Implantationsdauer verstellbar, und wird nicht durch anwachsendes Gewebe (Bindegewebe) blockiert. Die polierte Umfangsfläche kann auch durch eine andere Oberflächengestaltung erreicht sein, welche zu einer Verringerung von Anhaftungen führt. In Betracht kommt eine Anodisierung der Oberfläche, insbesondere bei Endoprothesen aus Titan.

Die Verstellmutter weist zweckmäßigerweise an ihrer Umfangsfläche eine Mehrzahl von Radiallöchern auf, die vorzugsweise einen gleichmäßigen Winkelabstand aufweisen. Diese Radiallöcher dienen zur Aufnahme eines Verstellstifts. Dieser wird in eines dieser Löcher eingesteckt, so kann die Verstellmutter um einen bestimmten Winkelbetrag verdreht werden, bis der Verstellstift seinen Anschlag erreicht. Durch Umstecken in eines der anderen, vorzugsweise winkelgleich angeordneten Radiallöcher kann der Verstellstift erneut betätigt werden, so dass im Ergebnis eine Verdrehung der Verstellmutter und damit eine Längenverstellung erreicht ist. Dies bietet auch den Vorzug einer Notbetätigung, falls die Längeneinstelleinrichtung mittels Verstellschlüssel nicht betätigt werden kann.

Die Verstellmutter weist zweckmäßiger Weise eine abgerundete Tastmarkierung auf. Damit ist es ermöglicht, eine "Nulllage" der Verstellmutter in Drehrichtung definiert festzulegen. Dies ist zweckmäßig bei einer rechnerischen Übertragung der nachzustellenden Länge bzw. des Längenwachstums, und zwar gemessen in Umdrehungen der Verstellmutter. Um hier eine Nullposition zu haben, ist die Tastmarkierung ein großer Vorteil. Zweckmäßigerweise weist der Außenstab, gegen dessen Stirnseite die Verstellmutter anliegt, eine formgleiche Fortsetzung der Tastmarkierung auf. Damit ergibt sich ein harmonischer Übergang zwischen der Tastmarkierung auf der Verstellmutter und der Fortsetzung auf dem Außenstab. Der Gefahr von Irritationen umliegenden Gewebes wird damit wirksam begegnet.

Mit Vorteil sind an dem Innenstab Vertiefungen vorgesehen, in die ein an dem Außenstab angeordnetes Verriegelungselement eingreift. Bei diesen Vertiefungen kann es sich um eine Reihe von Bohrungen handeln, die an der Außenseite des Schafts angeordnet sind. Zweckmäßigerweise sind sie in einer Axialnut angeordnet. Sie dienen dazu, dass eine Schraube eingedreht ist, welche mit ihrer Spitze in die Vertiefung eingreift und damit den Innenstab gegenüber einer ungewollten Bewegung in axialer Richtung sichert. Damit wird eine unbeabsichtigte Trennung von Innenstab und Außenstab sicher vermieden. Dieser kann damit entgegen gewirkt werden. Mit Vorteil ist die Befestigungsschraube als eine Madenschraube ausgeführt. Sie benötigt wenig Platz und kann dennoch eine ausreichend sichere Arretierung der Längeneinstelleinrichtung erreichen.

Vorzugsweise besteht mindestens eines der beiden Elemente, das Gewinde des Innenstabs und/oder das Innengewinde der Verstellmutter aus titanfreiem Material, insbesondere Cobalt-Chrom-Material. Es bietet den Vorzug, dass es gerade im Zusammenspiel mit - dem im Prothesenbau bevorzugten Material - Titan nicht zum Festgehen des Gewindes kommt. Ein Schutz gegenüber einer unbeabsichtigten Blockade des Gewindes insbesondere durch Festgehen ist ein erheblicher Vorteil für die erfindungsgemäße Röhrenknochenprothese, deren wichtigste Eigenschaft die Längsverschieblichkeit ist.
Die Erfindung wird nachfolgend unter Bezugnahme auf die beigefügte Zeichnung erläutert, in der vorteilhafte Ausführungsbeispiele dargestellt sind. Es zeigen:
- Fig. 1: eine Schnittansicht für eine Kniegelenkprothese gemäß einem ersten Ausführungsbeispiel der Erfindung;
- Fig. 2: eine Totalprothese auf Grundlage des ersten Ausführungsbeispiels gemäß Fig. 1 in Frontal und Lateralansicht;
- Fig. 3: Explosionsansichten zu Fig. 2;
- Fig. 4: eine Schnittansicht zu einer Variante;
- Fig. 5a-e: Darstellungen zur Durchführung einer Längenverstellung;
- Fig. 6: eine Explosionsansicht eines zweiten Ausführungsbeispiels der Erfindung;
- Fig. 7a-c: vergrößerte Detaildarstellungen zum zweiten Ausführungsbeispiel;
- Fig. 8: eine Funktionsdarstellung eine Längeneinstelleinrichtung;
- Fig. 9: eine Darstellung im montierten Zustand; und
- Fig. 10: eine perspektivische Ansicht.

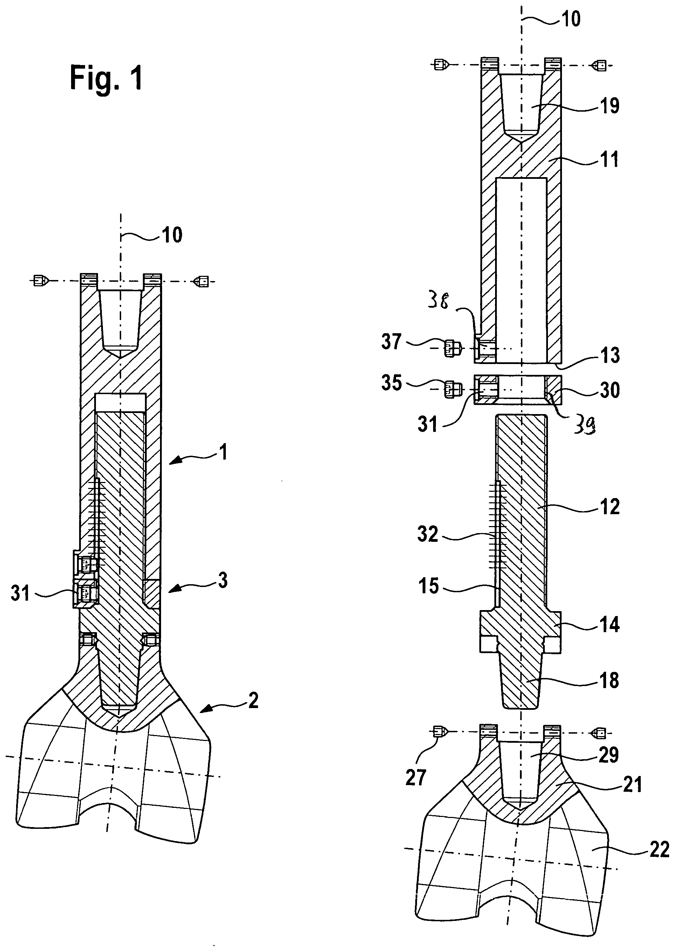

In Fig. 1 ist ein Ausführungsbeispiel der erfindungsgemäßen Prothese dargestellt, das als Gelenkprothese zum teilweisen Ersatz des Knies und eines Teils des distalen Femurs vorgesehen ist. Es umfasst als Komponenten einen Schaft 1, eine Gelenkeinrichtung 2 und eine Längeneinstelleinrichtung 3.

Der Schaft 1 umfasst einen Außenstab 11 und einen Innenstab 12, der teleskopartig längs seiner Mittelachse 10 verschieblich in dem Außenstab 11 geführt ist. Der Außenstab 12 weist an seinem ersten Ende einen weiblichen Konussteckverbinder 19 auf, der bei Bedarf zum Ankoppeln weiterer Stabsegmente (in Fig. 1 nicht dargestellt) dient; es sei angemerkt, dass der weibliche Konussteckverbinder auch durch einen Blindstopfen verschlossen sein kann. An seinem zweiten Ende weist der Außenstab einen Stirnflansch 13 mit einer radial ausgerichteten Stirnfläche auf. Der Innenstab 12 weist an seinem ersten Ende einen komplementären, männlichen Konussteckverbinder 18 auf, der zum Eingriff in einen passenden weiblichen Konussteckverbinder 29 an der Gelenkeinrichtung 2 ausgebildet ist. Am Übergang zu dem Konussteckverbinder 18 weist der Innenstab 12 einen Bund 14 auf, dessen eine, dem festen Ende zugewandte Stirnfläche als Anschlag für den Konussteckverbinder 18 und dessen andere, dem Schaft des Innenstabs 12 zugewandte Stirnfläche als Anschlag für eine Verstellmutter 30 fungiert.

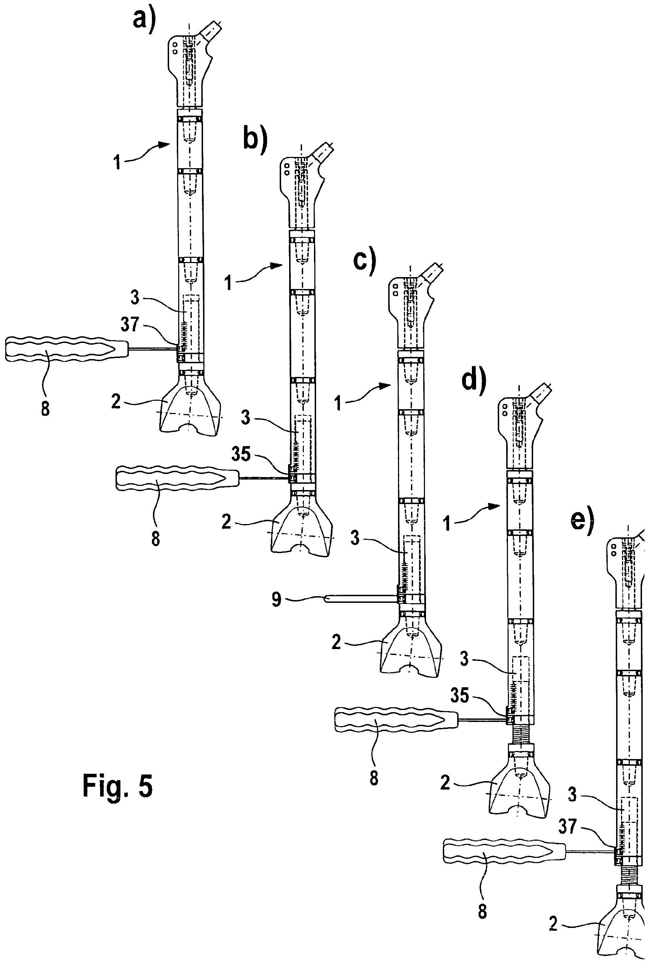

Die Längeneinstelleinrichtung 3 umfasst die Verstellmutter 30 mit einem eingängigen Innengewinde 39, das mit einem auf dem Innenstab 12 angeordneten eingängigen Stellgewinde 32 kämmt. Die Verstellmutter 30 weist an ihren Seitenflächen eine Mehrzahl von Eingriffsöffnungen 31 auf, die als Radialbohrungen ausgestaltet sind. Sie sind dazu ausgebildet, einen Stift 9 (s. Fig. 5c) als Betätigungselement aufzunehmen. Damit wird die Verstellmutter 30 gegenüber dem Innenstab 12 mit dem Gegengewinde 32 verdreht, wodurch die Verstellmutter 30 entlang der Mittelachse 10 sich bewegt. Die Verstellmutter 30 liegt in Grundstellung unmittelbar an dem Stirnflansch 13 des Außenstabs 11 an und nimmt diesen bei ihrer Bewegung mit. Dadurch bewegt sich der Außenstab 11 entlang der Längsachse 10 relativ zu dem Innenstab 12 derart, dass sich der Abstand zwischen Verstellmutter 30 und dem Bund 14 vergrößert und die Gesamtlänge des Schafts 1 zunimmt. Bei Verdrehung der Verstellmutter 30 in Gegenrichtung läuft der Vorgang in umgekehrter Richtung ab und die Gesamtlänge verkürzt sich.

Zur Fixierung einer eingestellten Länge sind Sicherungseinrichtungen vorgesehen. Sie umfassen eine Verstellsicherung 35 und eine Verdrehsicherung 37. Die Verstellsicherung 35 weist eine Klemmschraube auf, die in eine der radialen Bohrungen 31 der Verstellmutter 30 eingesetzt ist und mit ihrer Spitze auf eine Abflachung 15 am Außenstab 12 einwirkt. Damit wird eine formschlüssige Verriegelung der Verstellmutter 30 erreicht. Es besteht so Sicherheit, dass auch bei großer Last und häufigen Lastwechseln keine unbeabsichtigte Verdrehung der Verstellmutter 30 mit entsprechender Längenänderung entstehen kann. Die Verdrehsicherung 37 ist ähnlich aufgebaut und weist eine Fixierschraube auf, die in eine Radialbohrung im Bereich des Stirnflansch 13 am Außenstab angeordnet ist. Die Fixierschraube wirkt mit ihrer Spitze in den Bereich des Gegengewindes 32 und sichert damit den Außenstab 11 vor einer Verdrehung gegenüber dem Innenstab 12. Dieser ist wiederum über eine an sich bekannte Konussicherung 27 mittels zweier diametral gegenüberliegender Fixierschrauben an einer unerwünschten Verdrehung gegenüber der Gelenkeinrichtung 2 gehindert. Damit ergibt sich eine durchgehende Rotationssicherung von der Gelenkeinrichtung 2 über die Längeneinstelleinrichtung 3 bis zum Schaft 1.

Der Vorgang der Längenverstellung ist in Fig. 5 verdeutlicht am Beispiel einer implantierten Prothese, die zur Anpassung an das Wachstum des Patienten auf eine größere Länge eingestellt werden soll. Dazu ist es zuerst erforderlich, mittels minimal invasiver Chirurgie einen Zugang zur Prothese zu schaffen. In der Regel genügt hierfür eine Stichinzision. In einem ersten Schritt (Fig. 5a) wird ein Schraubendreher 8 durch die Inzision geführt und in Eingriff mit der Sicherungsschraube für die Rotationssicherung 37 gebracht. Die Rotationssicherung wird durch Herausdrehen der Schraube gelöst. In einem zweiten Schritt (Fig. 5b) wird die Verstellsicherung 35 auf dieselbe Weise gelöst. Damit ist die Längeneinstelleinrichtung 3 frei und kann betätigt werden. Der Schraubendreher 8 wird entfernt und ein Verstellstift 9 durch die Inzision eingeführt und in Eingriff mit einer der Radialbohrungen 31 der Verstellmutter gebracht. Durch Verschwenken des Stifts 9 wird die Verstellmutter 30 ein Stück gedreht, und der Stift 9 in eine benachbarte Radialbohrung eingesetzt und die Verstellmutter 30 wieder ein Stück gedreht. Bei dem dargestellten Ausführungsbeispiel ist die Gewindesteigung so gewählt, dass sich pro Umdrehung der Verstellmutter 30 eine Längenänderung um 2 mm ergibt. Ist die gewünschte Länge eingestellt, wird der Stift 9 entnommen und wiederum der Schraubendreher 8 eingeführt, um nacheinander die Verstellsicherung 35 (Fig. 5d) und die Verdrehsicherung (Fig. 5e) wieder einzusetzen und damit zu sichern.

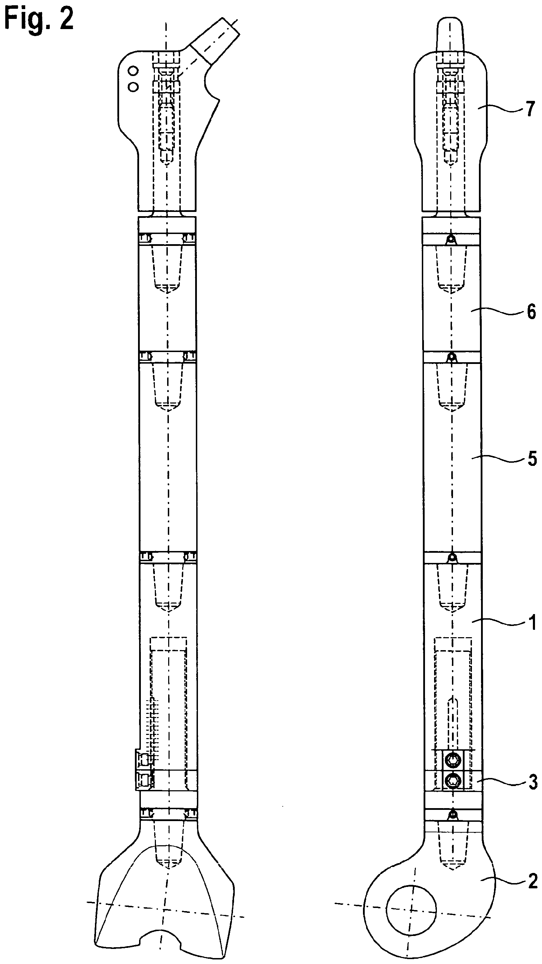

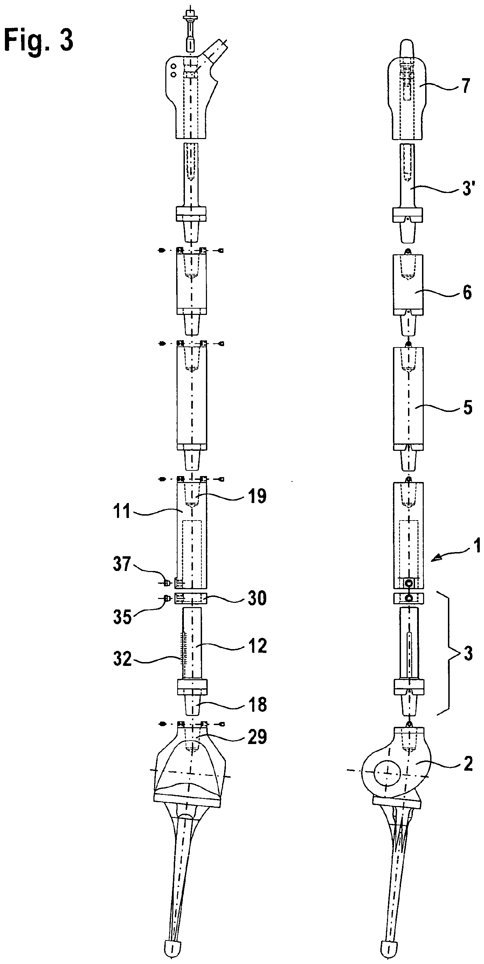

Bei der in Fig. 1 dargestellten Ausführungsform handelt es sich um eine Basis-Prothese. Sie kann mit zusätzlichen Elementen ergänzt werden, wie in den Fig. 2 und 3 dargestellt. Dort sind zusätzliche Schaftsegmente 5, 6 vorgesehen, die über Konussteckverbinder, die passend sind zu den Konussteckverbindern 18, 19 des Schafts 1 und 29 der Gelenkeinrichtung 2, zusammengesetzt sind zu einem langen Schaft (s. Explosionsdarstellung in Fig. 3). An dessen oberen Ende ist eine Femurhalsprothese 7 angeordnet. Damit ist eine Femurtotalprothese gebildet, die nicht nur wie im Stand der Technik durch Auswahl passender Schaftsegmente 5, 6 mit abgestufter Länge gebildet sein kann, sondern dank der modularen Längeneinstelleinrichtung 3 sogar stufenlos verstellbar ist. Damit kann eine Feinanpassung vorgenommen werden. Vorzugsweise hat der Schaft 1 mit der Längeneinstelleinrichtung 3 in einer Grundstellung (wie in Fig. 1 dargestellt) dieselbe Länge wie eines der Schaftsegmente, zum Beispiel das Schaftsegment 5. Damit ist ein Prothesensystem gebildet, bei dem je nach Bedarf ein längeneinstellbarer oder ein längenfester Schaft durch einfachen Austausch der Elemente 1, 5 gebildet werden kann.

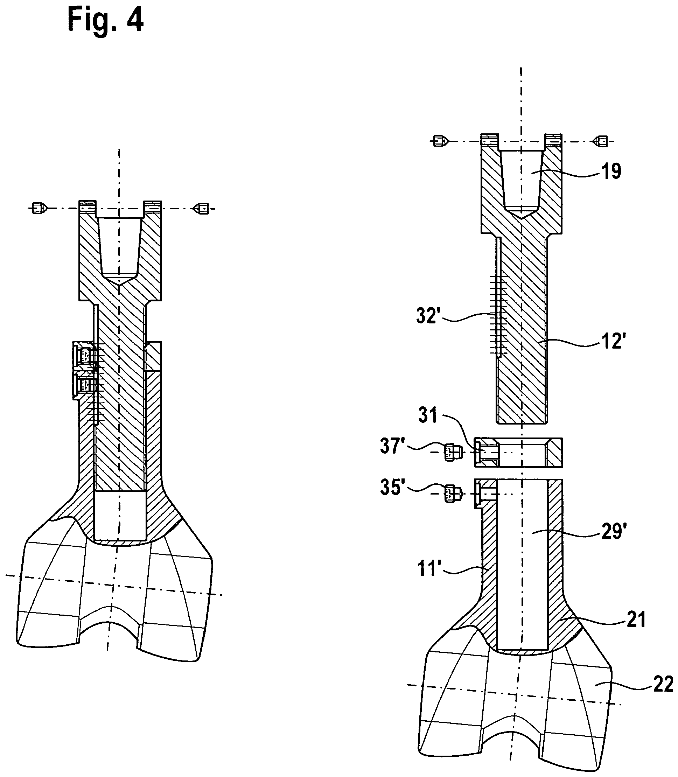

Eine Ausführungsvariante ist in Fig. 4 dargestellt, wobei identische Elemente mit denselben Bezugsziffern versehen sind. Der Unterschied zu dem ersten Ausführungsbeispiel besteht im Wesentlichen darin, dass die Anordnung des Außenstabs 11' und Innenstabs 12' invers ausgeführt ist, d. h. der Außenstab 11' ist an der Gelenkeinrichtung 2 angeordnet und der Innenstab 12' bildet das erste Ende mit der Konussteckverbindung 19. Eine solche invers ausgeführte Längeneinstelleinrichtung 3' kann auch am ersten Ende eines langen Schafts mit mehreren Schaftsegmenten 5, 6 vorgesehen sein, wie in Fig. 3 dargestellt.

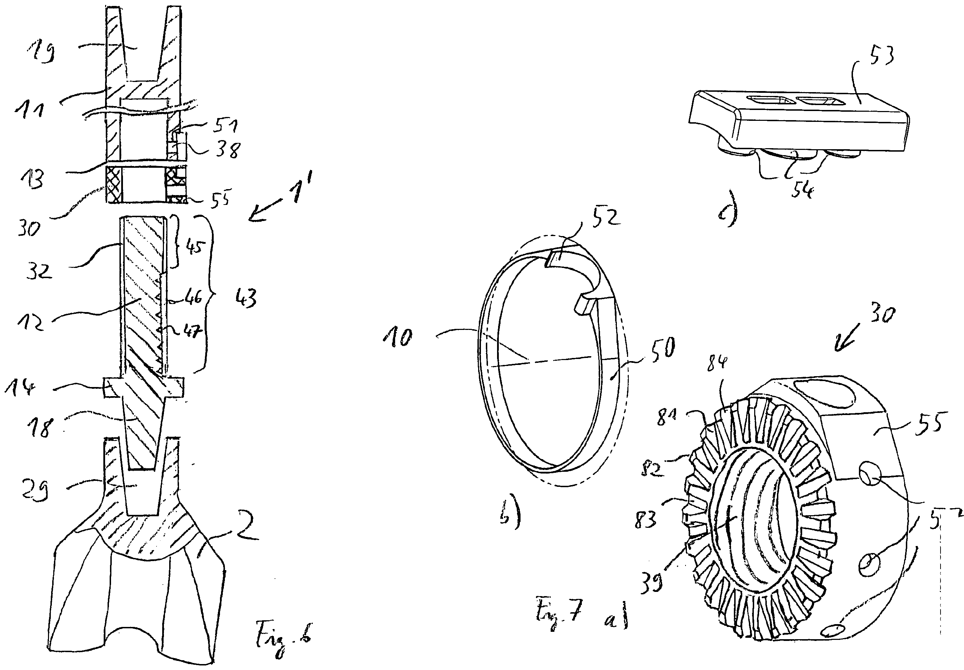

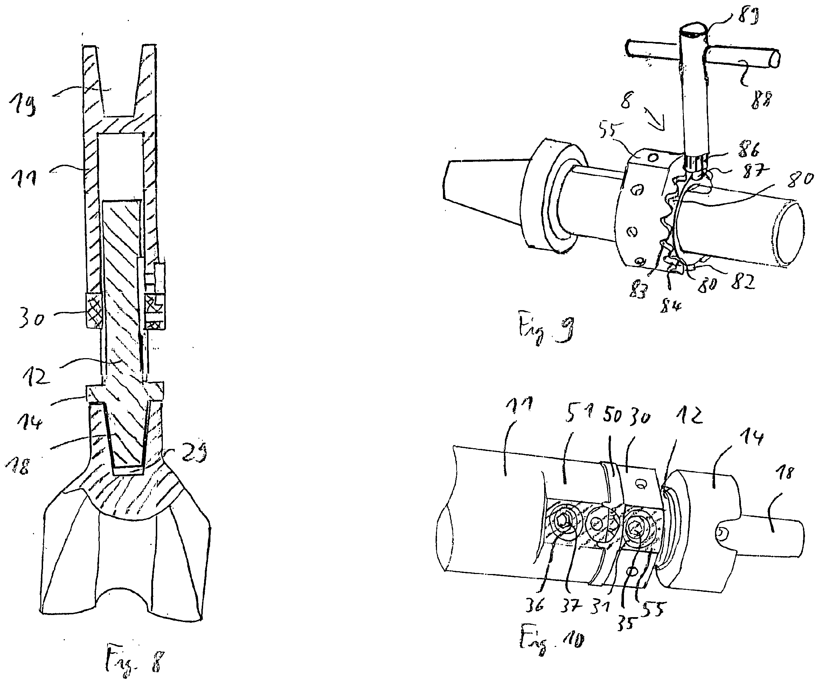

Es wird Bezug genommen auf das in den Fig. 6-10 dargestellte zweite Ausführungsbeispiel. Es weist eine besondere Betätigungseinrichtung für die Längeneinstelleinrichtung auf. Gleichartige Elemente sind mit denselben Bezugsziffern versehen. Von dem Bund 14 zum anderen, freien Ende des Schafts 12 erstreckt sich ein Einsteckbereich 43. Dieser ist mit dem Außengewinde 32 versehen. Es ist eingängig und die einzelnen Gewindegänge weisen eine Querschnittsform auf, die im Wesentlichen dreiecksförmig mit abgeflachter Spitze ist. Ferner weist der Innenstab bis auf einen kurzen, etwa dem 1,5-fachen des Stabdurchmessers entsprechenden Führungsabschnitt 45 eine Längsnut 46 auf, an deren Nutgrund eine Anzahl von Sacklöchern 47 in einer parallel zur Längsachse orientierten Linie ausgebildet ist.
An der Verstellmutter 30 (s. Fig. 7a) ist an ihrem im Wesentlichen glatten Außenmantel eine als Erhebung ausgeführte Tastmarkierung 55 angeordnet. Weiter sind am Außenmantel acht Senklöcher 57 in gleichmäßigem Winkelabstand in einer einheitlichen Radialebene angeordnet, von denen eines in der Tastmarkierung 55 angeordnet ist. An ihrem unteren, den Innenstab 12 zugewandten Rand weist die Verstellmutter 30 eine zu dem Bund 14 komplementäre Gestaltung mit einer planen äußeren Anlagefläche. Das Innengewinde 39 ist in einen aus Kobalt-Chrom (CoCr) hergestellten Bereich der Verstellmutter 30 angeordnet; vorzugsweise besteht die gesamte Verstellmutter 30 aus Kobalt-Chrom-Material.
Die Verstellmutter weist an ihrem oberen Rand eine umlaufende Verzahnung 81 auf, die Teil einer Betätigungseinrichtung 8 ist. Die Verzahnung 81 hat ein wellenförmiges Profil mit abgerundeten Spitzen 82 und Tälern 83. Die die Spitzen 82 und Täler 83 verbindenden Flanken 84 sind als Steilflanken ausgeführt und haben in ihrem Mittelteil eine Steigung (bezogen auf die durch den oberen Rand 56 definierte Radialebene) von 60 Grad. Die Täler 83 steigen von außen nach innen an, so dass sich eine kegelig zulaufendes Zahnstruktur ergibt, wie sie insbesondere für einen Winkeltrieb geeignet ist. Die Verzahnung 81 ist an einem an der Außenseite des oberen Rands 56 umlaufenden Rezess 80 so angeordnet, dass die Spitzen 82 nicht hinausragen, sondern bündig mit der durch den oberen Rand definierten Ebene abschließen (s. Fig. 9, aus Gründen der Übersichtlichkeit ist der Außenstab nicht dargestellt). Damit ergibt sich an dem oberen Rand eine Art zweischalige Struktur, mit einem umlaufenden inneren Ring, welcher eine plane und hinterschnittfreie Anlagefläche bildet, als innere Schale und der Verzahnung 81, deren abgerundete Spitzen 82 bündig auf demselben Niveau wie der innere Ring 56' abschließen, als äußere Schale.

Der Stirnflansch 13 des Außenstabs 11 ist im Wesentlichen plan, insbesondere ist er hinterschnittfrei, d. h. an keiner Stelle ist ein Hinterschnitt ausgebildet. Am Außenmantel des Außenstabs 11 ist benachbart zum Rand eine zweite Erhebung 51 angeordnet. Sie ist mit einer Radialbohrung 38 versehen, die als Lagersitz für einen Verstellschlüssel 89 der Betätigungseinrichtung 8 fungiert. Der Abstand zwischen der Radialbohrung 38 und dem Stirnflansch 13 ist auf die Abmessungen des Verstellschlüssel 89 abgestimmt, wie nachfolgend näher beschrieben ist.

An dem Außenstab 11 kann eine Madenschraube 37 als Verriegelungsinstrument vorgesehen sein. Vorzugsweise ist sie in die Lagerbohrung 38 einschraubbar und ragt mit ihrer Spitze im eingeschraubten Zustand in die Längsnut 46, genauer gesagt eines der Sacklöcher 47, und sichert den Innenstab 12 damit gegenüber unerwünschten Luxationsbewegungen.

Der Verstellschlüssel 89 ist aufgebaut wie ein Kegeltriebschlüssel, wie er zur Betätigung von Spannfuttern bekannt ist. Es weist an seinem hinteren Ende einen Betätigungsgriff 88 auf, wobei es sich im einfachsten Fall um eine Querstange handeln kann. Am vorderen Ende ist eine Kegelverzahnung 86 vorgesehen, die so beschaffen ist, um mit der Verzahnung 81 an der der Verstellmutter 30 zu kämmen. Um die Kegelverzahnung 86 in Eingriff mit der Verzahnung 81 zu bringen, ist an der vorderen Spitze ein Lagerzapfen 87 ausgebildet, der komplementär zu der Radialbohrung 38 ausgeführt ist, so dass ein Drehlager gebildet ist. Der Abstand der Radialbohrung 38 von dem Stirnflansch 13 ist so auf den Durchmesser der Kegelverzahnung 96 abgestimmt, dass bei in die Radialbohrung 38 eingestecktem Verstellschlüssel 89 die Kegelverzahnung 86 in Eingriff mit der Verzahnung 81 der Verstellmutter 30 ist, die bündig mit ihrem oberen Rand an dem Stirnflansch 13 des Außenstabs 11 anliegt.

Die Betätigungseinrichtung 8 wird wie folgt betätigt. Im Ausgangszustand ist die Verstellmutter 30 auf das Außengewinde 32 des Innenstabs 12 aufgeschraubt. Er ist soweit in den Außenstab 11 eingeschoben, bis die Verstellmutter 30 mit ihrem oberen Rand bündig an dem Stirnflansch 13 des Außenstabs 11 anliegt. Durch Drehen des in die Lagerbohrung 31 eingesteckten Verstellschlüssel 89 kämmt dessen Kegelverzahnung 86 mit der Verzahnung 81 der Verstellmutter 30, wodurch sich diese dreht und der Innenstab 12 aus dem Außenstab 11 herausgeschoben wird. Der Betrag der Schiebestrecke ist hierbei bestimmt durch die Steigung des mit der Verstellmutter 30 zusammenwirkenden Außengewindes 32 und dem Übersetzungsverhältnis zwischen Kegelverzahnung 86 und der Verzahnung 81. Bei der Verstellung bleibt die Verstellmutter 30 in Anlage an dem Außenstab 11.

Tritt aufgrund des Wachstums des Patienten (oder einer Längung stützender Bänder) eine Verlängerung des Oberschenkels auf, kann die erfindungsgemäße Endoprothese daran angepasst werden. Dies geschieht, indem die Verstellmutter 30 nachgestellt wird. Dazu braucht nur mittels eines kleinen und damit Patienten schonenden Eingriffs das Antriebswerkzeug 89 in die Lagerbohrung 38 eingesetzt zu werden, und durch Drehen wird die Verstellmutter 30 nachgestellt. Der Nachstellweg ist die durch die Anzahl der Umdrehungen des Verstellschlüssels 89 eindeutig bestimmt. Um die Anzahl der Umdrehungen leicht nachkontrollieren zu können, dient die Tastmarkierung 55 an der Verstellmutter 30. In der Ausgangslage fluchtet sie mit der gleichartigen Erhebung 51 an dem Außenstab 11, und steht immer dann, wenn die Verstellmutter 30 eine vollständige Drehung durchlaufen hat, wieder in fluchtender Position. Damit kann die korrekte Positionierung leicht durch Tasten auch von außen überprüft werden.

Um auch nach längerer Implantationszeit eine einwandfreie Funktion der Betätigungseinrichtung 8 zu gewährleisten, sind ein Verzahnungschutzring 50 mit einer Anformung 52 für die Kegelverzahnung 86 des Verstellschlüssels 89 und eine Multiabdeckung 53 mit mehreren (im dargestellten Ausführungsbeispiel drei) Zapfenstumpfen 54 vorgesehen (s. Fig 7b, c). Der Verzahnungsschutzring ist zwischen der Verstellmutter 30 und dem Stirnflansch 13 des Außenstabs 11 angeordnet und deckt die Verzahnung 81 nach außen ab. Damit wird ein Einwachsen von Gewebe in die Verzahnung mit der damit einhergehenden Gefahr eines Blockierens verhindert. Um weiter ein Einwachsen in die radialen Bohrungen 31, 38 zu verhindern, ist die Multiabdeckung 53 vorgesehen. Sie ist ein im Wesentlichen quaderförmiger Block und ist mit ihren Zapfenstumpfen 54 in die radialen Bohrungen 31, 38 eingesetzt und durch Klemmung gehalten. Sie deckt den in Fig. 10 schraffiert dargestellten Bereich ab und verhindert so zuverlässig ein unerwünschtes Einwachsen von Gewebe. Zur Längenverstellung braucht sie nur abgenommen zu werden, und der Zugang zu den Schrauben 35, 37 sowie zur Verzahnung 81 ist frei.

Bei der in Fig. 10 dargestellten Variante ist zur Aufnahme für den Lagerzapfen 87 des Verstellschlüssels 89 eine eigenständige Bohrung vorgesehen, die gesondert ist von der Bohrung, in welcher die Schraube 37 zur Verdrehsicherung aufgenommen ist (statt der kombinierten Ausführung, wie in den Fig. 1 - 8 dargestellt).

## Patentansprüche

1. Prothese zum mindestens teilweisen Ersatz eines Röhrenknochens und eines angrenzenden Gelenks, umfassend
einen länglich gestreckten Schaft (1) mit einem ersten und einem zweiten Ende, sowie
eine Gelenkeinrichtung (2), die am zweiten Ende des Schafts (1) angeordnet ist,
wobei der Schaft (1) und die Gelenkeinrichtung (2) über komplementäre Konussteckverbinder (18, 29) gekoppelt sind,
der Schaft einen Innenstab (12) und einen koaxialen Außenstab (11) aufweist,
eine Längeneinstelleinrichtung (3) vorgesehen ist, welche den Schaft (1) längs seiner Achse (10) teleskopartig betätigt und auf den Innenstab (12) und den Außenstab (11) wirkt, wobei
die Längeneinstelleinrichtung (3) modular ausgeführt ist und an ihrem proximalen und distalen Ende mit komplementären Konussteckverbindern (18, 19) versehen ist, wobei
die Längeneinstelleinrichtung ferner mit einer formschlüssig wirkenden Verdrehsicherung (37) versehen ist, **dadurch gekennzeichnet, dass** an dem einen der beiden Enden der Längeneinstelleinrichtung (3) ein männlicher und an dem anderen der beiden Enden ein weiblicher Konussteckverbinder vorgesehen ist, dass die Verdrehsicherung (37) am Aussenstab (11) angeordnet ist und in eine Längsausnehmung (15) am Innenstab (12) eingreift, und dass
der Innenstab (12) mittels zweier diametral gegenüberliegender Fixierschrauben an einer Verdrehung gegenüber der Gelenkeinrichtung (2) gehindert wird.

2. Prothese nach Anspruch 1, **dadurch gekennzeichnet, dass** eine Doppelsicherung vorgesehen ist, welche zusätzlich zur Verdrehsicherung (37) eine Verstellsicherung (35) umfasst.

3. Prothese nach Anspruch 2, **dadurch gekennzeichnet, dass** die Verdrehsicherung (37) drehfest und die Verstellsicherung (35) drehbeweglich angeordnet sind.

4. Prothese nach einem der vorangehenden Ansprüche, **dadurch gekennzeichnet, dass** ein Druckflansch (15) vorgesehen ist, der zwei einander gegenüberliegende Bundflächen aufweist, von denen die eine ein Schublager für die Steckverbinder und die andere einen Anschlag für die Längenverstellung bildet.

5. Prothese nach einem der vorangehenden Ansprüche, **dadurch gekennzeichnet, dass** eine zweite Längeneinstelleinrichtung (3') für den Schaft (1) vorgesehen ist.

6. Prothese nach einem der vorangehenden Ansprüche, **dadurch gekennzeichnet, dass** die Längenstelleinrichtung (3) einen in Grundstellung gekapselten Schraubtrieb (30, 32) aufweist.

7. Prothese nach einem der vorangehenden Ansprüche, **dadurch gekennzeichnet, dass** eine Betätigungseinrichtung (8) für die Längeneinstelleinrichtung (3) mit einem Gewinde (32) auf dem Innenstab (12) und einer auf das Gewinde (32) geschraubten Verstellmutter (30) mit einer umlaufenden Verzahnung (81) vorgesehen ist, wobei am Außenstab (11) eine Lagerbohrung (38) für einen in die umlaufende Verzahnung (81) greifenden Verstellschlüssel (89) vorgesehen ist.

8. Prothese nach Anspruch 7, **dadurch gekennzeichnet, dass** die Verstellmutter (30) mit ihrem oberen Rand abhebbar auf einem Stirnflansch (13) des Außenstabs (11) auf liegt und hinterschnittfrei mit ihm zusammenwirkt.

9. Prothese nach Anspruch 7 oder 8, **dadurch gekennzeichnet, dass** die umlaufende Verzahnung (81) als Steilverahnung ausgeführt ist, deren lasttragenden Flanken (84) einen Flankenwinkel von mindestens 50 Grad bis höchstens 85 Grad, vorzugsweise mindestens 60 Grad aufweisen.

10. Prothese nach Anspruch 7, 8 oder 9, **dadurch gekennzeichnet, dass** die Verzahnung (81) in einem umlaufenden Rezess (80) angeordnet ist.

11. Prothese nach einem der vorangehenden Ansprüche, **dadurch gekennzeichnet, dass** ein Schutzring (50) aus vorzugsweise elastischem Kunststoffmaterial vorgesehen ist, der zwischen Verstellmutter (30) und Stirnflansch (13) angeordnet die Verzahnung (81) nach außen abdeckt.

12. Prothese nach einem der vorangehenden Ansprüche, **dadurch gekennzeichnet, dass** das Innengewinde (39) der Verstellmutter (30) eingängig ist.

13. Prothese nach einem der vorangehenden Ansprüche, **dadurch gekennzeichnet, dass** das Gewinde (32) des Innenstabs (12) geplant ist.

14. Prothese nach einem der vorangehenden Ansprüche, **dadurch gekennzeichnet, dass** die Verstellmutter (30) eine polierte Mantelfläche aufweist.

15. Prothese nach einem der vorangehenden Ansprüche, **dadurch gekennzeichnet, dass** die Verstellmutter (30) eine Mehrzahl von Radiallöchern (57) in vorzugsweise gleichmäßigen Winkelabstand an ihrer Mantelfläche aufweist.

16. Prothese nach einem der vorangehenden Ansprüche, **dadurch gekennzeichnet, dass** die Verstellmutter (30) eine abgerundete Tastmarkierung (55) aufweist, die vorzugsweise formgleich an dem Außenstab (11) fortgesetzt ist.

17. Prothese nach einem der vorangehenden Ansprüche, **dadurch gekennzeichnet, dass** am Innenstab (12) eine Mehrzahl von Vertiefungen (47) vorgesehen sind, in die ein an dem Außenstab (11) angeordnetes Verriegelungselement (37) eingreift.

18. Prothese nach einem der vorangehenden Ansprüche, da durch gekennzeichnet, dass das Gewinde (32) und/oder das Innengewinde (39) aus titanfreiem Material, insbesondere Kobaltchrom-Material, bestehen.

19. Prothesensystem umfassend eine Prothese nach einem der vorangehenden Ansprüche und mehreren verschieden langen Schaftelementen (5, 6), die über die Steckverbinder koppelbar sind.

20. Prothesensystem nach Anspruch 19, **dadurch gekennzeichnet, dass** eines der Schaftelemente (5, 6) dieselbe Länge wie die Längeneinstelleinrichtung (3) in ihrer Grundstellung aufweist.

## Claims

1. Prosthesis for at least partial replacement of a tubular bone and of an adjoining joint, comprising
an elongate shaft (1) with a first end and a second end, and
a joint mechanism (2) arranged at the second end of the shaft (1),
wherein the shaft (1) and the joint mechanism (2) are coupled via complementary conical plug connectors (18, 29),
the shaft has an inner rod (12) and a coaxial outer rod (11),
a length-adjusting mechanism (3) is provided which actuates the shaft (1) along its axis (10) in the manner of a telescope and acts on the inner rod (12) and the outer rod (11), wherein
the length-adjusting mechanism (3) is of a modular design and is provided, at its proximal and its distal end, with complementary conical plug connectors (18, 19), wherein
the length-adjusting mechanism is further provided with an anti-rotation means (37) acting in form-fit engagement,
**characterized in that** a male conical plug connector is provided at one of the two ends of the length-adjusting mechanism (3) and a female conical plug connector is provided at the other of the two ends, **in that** the anti-rotation means (37) is arranged on the outer rod (11) and engages in a longitudinal recess (15) on the inner rod (12), and **in that**
the inner rod (12) is prevented from rotation with respect to the joint mechanism (2) by means of two radially opposite fixing screws.

2. Prosthesis according to Claim 1, **characterized in that** a dual securing mechanism is provided which comprises an adjustment lock (35) in addition to the anti-rotation means (37).

3. Prosthesis according to Claim 2, **characterized in that** the anti-rotation means (37) is fixed in rotation, and the adjustment lock (35) is arranged rotatably.

4. Prosthesis according to one of the preceding claims, **characterized in that** a compression flange (15) is provided which has two opposite collar faces, one of which is a thrust bearing for the plug connectors, and the other forms a stop for the length adjustment.

5. Prosthesis according to one of the preceding claims, **characterized in that** a second length-adjusting mechanism (3') is provided for the shaft (1).

6. Prosthesis according to one of the preceding claims, **characterized in that** the length-adjusting mechanism (3) has a screw drive (30, 32) which is encapsulated in the starting position.

7. Prosthesis according to one of the preceding claims, **characterized in that** an actuation mechanism (8) for the length-adjusting mechanism (3) having a thread (32) on the inner rod (12) and an adjustment nut (30) screwed onto the thread (32) is provided with a circumferential toothing (81), wherein a bearing bore (38) is provided on the outer rod (11) for an adjusting wrench (89) engaging in the circumferential toothing (81).

8. Prosthesis according to Claim 7, **characterized in that** the adjustment nut (30) lies with its upper edge liftably on a front flange (13) of the outer rod (11) and cooperates therewith without undercut.

9. Prosthesis according to Claim 7 or 8, **characterized in that** the circumferential toothing (81) is designed as a steep toothing, the load-bearing flanks (84) of which have a flank angle of at least 50 degrees to at most 85 degrees, preferably at least 60 degrees.

10. Prosthesis according to Claim 7, 8 or 9, **characterized in that** the toothing (81) is arranged in a circumferential recess (80).

11. Prosthesis according to one of the preceding claims, **characterized in that** a protective ring (50), preferably made of resilient plastic, is provided which is arranged between adjustment nut (30) and front flange (13) and covers the toothing (81) on the outside.

12. Prosthesis according to one of the preceding claims, **characterized in that** the internal thread (39) of the adjustment nut (30) is a single-start thread.

13. Prosthesis according to one of the preceding claims, **characterized in that** the thread (32) of the inner rod (12) is flattened.

14. Prosthesis according to one of the preceding claims, **characterized in that** the adjustment nut (30) has a polished jacket face.

15. Prosthesis according to one of the preceding claims, **characterized in that** the adjustment nut (30) has on its jacket surface a plurality of radial holes (57), preferably at uniform angular intervals.

16. Prosthesis according to one of the preceding claims, **characterized in that** the adjustment nut (30) has a rounded tactile marking (55) which is continued, preferably identically in shape, on the outer rod (11).

17. Prosthesis according to one of the preceding claims, **characterized in that** a plurality of depressions (47) are provided on the inner rod (12), into which depressions (47) a locking element (37) arranged on the outer rod (11) engages.

18. Prosthesis according to one of the preceding claims, **characterized in that** the thread (32) and/or the internal thread (39) are composed of titanium-free material, particularly cobalt-chromium material.

19. Prosthesis system comprising a prosthesis, according to one of the preceding claims, and a plurality of shaft elements (5, 6) which are of different lengths and can be coupled via the plug connectors.

20. Prosthesis system according to Claim 19, **characterized in that** one of the shaft elements (5, 6) is the same length as the length-adjusting mechanism (3) in its starting position.

## Revendications

1. Prothèse pour le remplacement au moins partiel d'un os long et d'une articulation adjacente, comprenant
une tige étirée longitudinalement (1) avec une première extrémité et une deuxième extrémité, ainsi que
un système d'articulation (2), qui est agencé à la deuxième extrémité de la tige (1),
dans laquelle la tige (1) et le système d'articulation (2) sont couplés par des connecteurs coniques complémentaires (18, 29),
la tige présente une barre intérieure (12) et une barre extérieure coaxiale (11),
il est prévu un système de réglage de longueur (3), qui actionne de façon télescopique la tige (1) le long de son axe (10) et qui agit sur la barre intérieure (12) et sur la barre extérieure (11),
dans laquelle le système de réglage de longueur (3) est réalisé de façon modulaire et est muni à son extrémité proximale et à son extrémité distale de connecteurs coniques complémentaires (18, 19),
dans laquelle le système de réglage de longueur est en outre muni d'un blocage de rotation (37) agissant par emboîtement,
**caractérisée en ce qu'**il est prévu un connecteur conique mâle à une première des deux extrémités du système de réglage de longueur (3) et un connecteur conique femelle à l'autre des deux extrémités, **en ce que** le blocage de rotation (37) est disposé sur la barre extérieure (11) et s'engage dans un évidement longitudinal (15) sur la barre intérieure (12) et **en ce que** la barre intérieure (12) est empêchée de tourner par rapport au système d'articulation (2) au moyen de deux vis de fixation diamétralement opposées.

2. Prothèse selon la revendication 1, **caractérisée en ce qu'**il est prévu un double blocage, qui comprend un blocage de déplacement (35) en plus du blocage de rotation (37).

3. Prothèse selon la revendication 2, **caractérisée en ce que** le blocage de rotation (37) est bloqué en rotation et le blocage de déplacement (35) est mobile en rotation.

4. Prothèse selon l'une quelconque des revendications précédentes, **caractérisée en ce qu'**il est prévu une aile de pression (15), qui présente deux faces extérieures opposées l'une à l'autre, dont l'une est un appui glissant pour les connecteurs et l'autre forme une butée pour le déplacement longitudinal.

5. Prothèse selon l'une quelconque des revendications précédentes, **caractérisée en ce qu'**il est prévu un deuxième système de réglage de longueur (3') pour la tige (1).

6. Prothèse selon l'une quelconque des revendications précédentes, **caractérisée en ce que** le système de réglage de longueur (3) présente un mécanisme à vis (30, 32) encapsulé en position de base.

7. Prothèse selon l'une quelconque des revendications précédentes, **caractérisée en ce qu'**il est prévu un système d'actionnement (8) pour le système de réglage de longueur (3) avec un filet (32) sur la barre intérieure (12) et un écrou de réglage (30) avec une denture périphérique (81) vissé sur le filet (32), dans laquelle il est prévu sur la barre extérieure (11) un alésage de palier (38) pour une clé de réglage (89) s'engageant dans la denture périphérique (81).

8. Prothèse selon la revendication 7, **caractérisée en ce que** l'écrou de réglage (30) repose avec son bord supérieur en pouvant être soulevé sur une bride frontale (13) de la barre extérieure (11) et coopère avec celle-ci sans contre-dépouille.

9. Prothèse selon la revendication 7 ou 8, **caractérisée en ce que** la denture périphérique (81) est réalisé sous forme de denture droite, dont les flancs qui supportent la charge (84) présentent un angle de flanc d'au moins 50 degrés à au plus 85 degrés, de préférence d'au moins 60 degrés.

10. Prothèse selon la revendication 7, 8 ou 9, **caractérisée en ce que** la denture (81) est disposée dans un creux périphérique (80).

11. Prothèse selon l'une quelconque des revendications précédentes, **caractérisée en ce qu'**il est prévu une bague de protection (50) en une matière plastique de préférence élastique, qui est disposée entre l'écrou de réglage (30) et la bride frontale (13) et recouvre la denture (81) vers l'extérieur.

12. Prothèse selon l'une quelconque des revendications précédentes, **caractérisée en ce que** le filet intérieur (39) de l'écrou de réglage (30) présente un filet unique.

13. Prothèse selon l'une quelconque des revendications précédentes, **caractérisée en ce que** le filet (32) de la barre intérieure (12) est aplani.

14. Prothèse selon l'une quelconque des revendications précédentes, **caractérisée en ce que** l'écrou de réglage (30) présente une surface latérale polie.

15. Prothèse selon l'une quelconque des revendications précédentes, **caractérisée en ce que** l'écrou de réglage (30) présente dans sa surface latérale une multiplicité de trous radiaux (57) à une distance angulaire de préférence uniforme.

16. Prothèse selon l'une quelconque des revendications précédentes, **caractérisée en ce que** l'écrou de réglage (30) présente un marquage de prise arrondi (55), qui est prolongé de préférence sous la même forme sur la barre extérieure (11).

17. Prothèse selon l'une quelconque des revendications précédentes, **caractérisée en ce qu'**il est prévu sur la barre intérieure (12) une multiplicité de creux (47), dans lesquels s'engage un élément de verrouillage (37) agencé sur la barre extérieure (11).

18. Prothèse selon l'une quelconque des revendications précédentes, **caractérisée en ce que** le filet (32) et/ou le filet intérieur (39) se composent d'un matériau sans titane, en particulier d'un matériau au cobalt-chrome.

19. Système de prothèse comprenant une prothèse selon l'une quelconque des revendications précédentes et plusieurs éléments de tige de longueur différente (5, 6), qui peuvent être couplés par les connecteurs.

20. Système de prothèse selon la revendication 19, **caractérisé en ce qu'**un des éléments de tige (5, 6) présente la même longueur que le système de réglage de longueur (3) dans sa position de base.
